# EUROPEAN PATENT APPLICATION

(11) **EP 1 277 719 A1**
(43) Date of publication of application: **22.01.2003**
(21) Application number: 02014804.5
(22) Date of filing: 02.07.2002
(51) Int. Cl.: C07C 11/02, C07C 1/20, C07C 1/24

(54) **Process for producing alpha-olefin**

(30) Priority: 16.07.2001 JP 2001215537
(71) Applicant: Kuraray Co., Ltd., Kurashiki-City, Okayama 710-8622 (JP)
(72) Inventor: Fuji, Junichi, Kashima-gun, Ibaraki (JP); Nakayama, Osamu, Kashima-gun, Ibaraki (JP); Onishi, Takashi, Chuo-ku, Tokyo (JP)
(74) Representative: Müller-Boré & Partner Patentanwälte

(57) **Abstract**

A production process for α-olefins making use of elimination reaction of a primary alcohol or ether is characterized by use of an alumina catalyst in the presence of an amine.

## Description

The present invention relates to a process for producing an α-olefin from a primary alcohol or an ether. α-Olefins provided by the present invention are useful as a material for polyolefins or as a material for plasticizers.

It has been known that α-olefins can be produced through elimination reactions of primary alcohols or ethers. In most of such reaction systems, however, internal olefins, isomers of the α-olefin of interest, are produced along with the intended α-olefin. These internal olefins are difficult to separate from the α-olefin of interest and pose problems in preparing an α-olefin at a high purity.

In a description of one known technique for producing an α-olefin from a primary alcohol or an ether at a relatively high purity, five different types of γ-alumina are used to synthesize 1-butene from n-butanol (J. Am. Chem. Soc., vol.83, pp.2847-2852(1961), referred to as Article 1, hereinafter). It has turned out that when the γ-alumina containing a larger amount of base components is used, the proportion of 1-butene in the entire butene products can be as high as 97.3%. It has also proven that the lower the reaction temperature is, the higher the proportion of 1-butene becomes. In another technique disclosed in US patent No. 4,234,752 (referred to as Article 2, hereinafter), a primary alcohol is allowed to undergo dehydration reaction at 200 to 500°C in the presence of inert gas using a base-treated γ-alumina as a catalyst. Specifically, the γ-alumina is immersed in an aqueous solution of a base such as sodium hydroxide or potassium hydroxide to neutralize acidic sites on the surface of the γ-alumina. In this manner, the proportion of the α-olefin with respect to the entire olefin products is improved.

In order to allow for the production of α-olefins in an industrially advantageous manner by taking advantage of elimination reaction of primary alcohols or ethers, it is important that the following three conditions be met together:
(a) In addition to the α-olefin of interest, internal olefin isomers can be produced in elimination reactions of primary alcohols or ethers. Due to the slight difference between the boiling point of internal olefins and that of the α-olefin, a considerable amount of α-olefin is lost during distillation process for increasing the purity of the α-olefin. This loss becomes significantly large and unnegligible in terms of cost efficiency as the yields of internal olefins are increased. Thus, the α-olefin of interest must be obtained with a high selectivity and purity.
(b) The catalyst used must maintain its reactivity and selectivity of the products for a prolonged time.
(c) Resulting industrial waste must be reduced to minimize cost and adverse effects to environment.

In view of these conditions, the above-described approaches described in Articles 1 and 2 are each accompanied by the following drawbacks.

Regarding the condition (a) above, the techniques of Articles (1) and (2) each yield the intended α-olefin at a proportion of approximately 97% with respect to the entire olefins and thus, a significant loss of α-olefin results during the separation process to increase the purity of the α-olefin.

Regarding the condition (b), the technique of Article (1) employs as a catalyst a γ-alumina obtained by mixing a potassium component upon its preparation, whereas the catalyst used in the technique of Article (2) is a γ-alumina treated with a base. Specifically, the γ-alumina is obtained by first immersing it in an aqueous solution of an alkali metal hydroxide such as potassium hydroxide, followed by washing and drying. In either process, an alkali metal hydroxide is used to neutralize the acidic sites on the surface of γ-alumina, thereby increasing the proportion of α-olefin with respect to the entire olefins produced. These catalysts, however, are susceptible to change on surfaces when the alkali metal components elute during the reaction. This change in the surface condition of the catalyst affects the reactivity and the selectivity of the products in the technique of Article (1). The reactivity and the selectivity of the products are also affected in the technique of Article (2), then, the reaction must be stopped to re-treat the catalyst with a base such as an alkali metal hydroxide. Such a retreatment process must be performed periodically during a long-term continuous reaction. Therefore, neither of the techniques described in Articles (1) and (2) provides sufficient activity, selectivity, or catalyst life required for catalysts for industrial use.

Regarding the condition (c) above, each of the techniques described in Articles (1) and (2) involves the use of an alkali metal hydroxide upon preparation or pretreatment of the catalyst and thus produces basic waste solution. This waste solution must be neutralized with an acid for disposal, and as a result, the volume of the waste solution is significantly increased.

As described, a need exists to further improve the conventional production processes of α-olefins that make use of elimination reactions of primary alcohols or ethers.

Accordingly, it is an objective of the present invention to provide an industrially advantageous, environmentally less harmful production process of α-olefins that takes advantage of elimination reaction of a primary alcohol or an ether in producing an α-olefin. Not only can this process produce an α-olefin at a high proportion with respect to the entire olefin products, but can also ensure the reactivity of the catalyst and the selectivity of the products for a prolonged time.

In an effort to find a way to solve the above-identified problems, the present inventors have found that, in producing an α-olefin from a primary alcohol or an ether, a higher proportion of the α-olefin can be obtained with respect to the entire olefin products and the reactivity of the catalyst and selectivity of the products can be maintained for a prolonged time by using an alumina catalyst in the presence of an amine. The present inventors have also found that the proportion of α-olefin to the entire olefin products can also be maintained by separating and collecting an ether compound, the intermediate produced during the reaction, along with unreacted materials, from the reaction products, and allowing them to undergo the reaction again. In this manner, the reaction progresses in the same fashion to produce a similarly high proportion of α-olefin with respect to the entire olefin products. These findings collectively led the present inventors to complete the present invention.

Accordingly, one aspect of the present invention provides a process for producing an α-olefin through elimination reaction of a primary alcohol or an ether, characterized in that an alumina catalyst is used in the presence of an amine.

In one embodiment of the process of the present invention, an ether intermediate produced in the reaction, along with unreacted primary alcohol or ether, is separated and collected from reaction products, and at least part of the collected ether intermediate, and unreacted primary alcohol or ether is returned to the reaction system.

Preferably, the amine is one having a boiling point higher than that of the α-olefin product by as much as 30°C or more under atmospheric pressure.

A primary alcohol or an ether for use in the present invention has a structure shown by the following formula (I):

R¹-O-R² (I)

wherein R¹ represents a straight or branched alkyl group having 3 to 20 carbon atoms, and R² represents a hydrogen atom, or a straight or branched alkyl group having 1 to 20 carbon atoms.

Examples of the straight or branched alkyl group having 3 to 20 carbon atoms as represented by R¹ in the above formula include n-propyl, n-butyl, isobutyl, n-pentyl, isopentyl, n-hexyl, n-heptyl, n-octyl, n-nonyl, n-decyl, undecyl, dodecyl, tetradecyl, hexadecyl and octadecyl. Examples of the straight or branched alkyl group having 1 to 20 carbons as represented by R² include methyl, ethyl, n-propyl, n-butyl, isobutyl, n-pentyl, isopentyl, n-hexyl, n-heptyl, n-octyl, n-nonyl, n-decyl, undecyl, dodecyl, tetradecyl, hexadecyl and octadecyl.

Examples of the primary alcohol include n-butanol, n-pentanol, 3-methyl-1-butanol, n-hexanol, n-octanol, n-nonanol, n-decanol, n-undecanol, n-dodecanol, n-tetradecanol and n-hexadecanol. Of these, n-hexanol, n-octanol, n-nonanol and n-decanol are preferred. Examples of the ether include methyl pentyl ether, hexyl methyl ether, methyl octyl ether, methyl nonyl ether, decyl methyl ether, ethyl pentyl ether, ethyl hexyl ether, ethyl octyl ether, methyl nonyl ether, decyl methyl ether, isopropyl pentyl ether, hexyl isopropyl ether, isopropyl octyl ether, isopropyl nonyl ether, decyl isopropyl ether, dihexyl ether, dioctyl ether and didecyl ether.

An alumina catalyst for use in the present invention may be any industrially produced, commercially available alumina, including γ-alumina, Al₂O₃-SiO₂, Al₂O₃-Cr₂O₃, Al₂O₃-ZrO₂ and Al₂O₃-TiO₂. Of these aluminas, γ-alumina is particularly suitable to serve as the alumina catalyst in the process of the present invention. The alumina catalyst may contain a small amount of an alkali metal, such as lithium and potassium, an alkaline earth metal, such as magnesium and barium, and a rare earth metal, such as lanthanum. No particular limitation is imposed on the shape and size of the alumina catalyst: the alumina catalyst of any size and shape, including granule, pellet, and cylindrical shape, may be used depending on the shape or the size of the reactor.

Examples of an amine for use with the alumina catalyst in the process of the present invention include, but are not limited to, primary amines such as butylamine, hexylamine and octylamine; secondary amines such as dibutylamine, dihexylamine and dioctylamine; tertiary amines such as tributylamine, triethylenediamine, tetramethylethylenediamine and N,N,N',N'-tetramethyl-1,6-hexamethylenediamine; and cyclic amines such as 1,8-diazabicyclo[5.4.0]undeca-7-ene, pyridine and dimethylaminopyridine.

While amine may be used in any amount, it is typically used in an amount of 0.1 to 20% by mass, and more preferably, in an amount of 0.1 to 5% by mass with respect to the primary alcohol or the ether used as the material.

The reaction process of the present invention may be carried out by using any known technique, including batch reaction process and continuous reaction process. Of these, continuous reaction process using a fixed bed reactor is preferred in view of its simple operation and productivity. Preferably, the fixed bed reactor is an ordinary, single- or multi-tubular gas-phase fixed bed reactor.

While the process of the present invention may be carried out at any reaction temperature, it is preferably carried out at a temperature of 150°C to 350°C and more preferably at 200°C to 320°C in order to maintain the reaction in gas phase. Also, the process of the present invention may be carried out under any reaction pressure. The reaction pressure is properly determined depending on the type of the material in order to maintain the reaction in gas phase and is preferably in the range of 3 to 500kPa, and more preferably in the range of 30 to 300kPa.

Although the amine, and the primary alcohol or ether may be simultaneously fed to the reactor filled with the alumina catalyst through independent routes, it is preferred in view of simple operation and construction that the amine be first mixed with the primary alcohol or the ether serving as the material to form a mixture, which in turn is fed to the reactor filled with the alumina catalyst. The rate at which the mixture is fed to the reactor is typically in the range of 0.1 to 50hr⁻¹, and preferably in the range of 0.5 to 10hr⁻¹ as measured in the liquid hourly space velocity (LHSV) in order to maintain the proportion of the α-olefin in the entire olefins in the reaction products at a sufficiently high value, as required, for example, for the material of polyolefins, although the rate may vary depending on the volume filled with the alumina catalyst, the type of the primary alcohol or the ether, reaction temperature, reaction pressure or other parameters.

In the process of the present invention, an inert gas, such as helium or argon, may be fed along with the amine and the primary alcohol or the ether serving as the material. In such a case, the amount of the inert gas to be fed is preferably in the range of 0.1 to 1000L per 1kg of the material (*i.e.*, primary alcohol or ether), and more preferably in the range of 0.5 to 300L per 1kg of the material.

The reaction mixture so obtained is subjected to simple distillation process to separate the intended α-olefin, other olefins including internal olefins, water, and other components, and if necessary, the α-olefin fraction is further purified using techniques such as distillation to obtain the α-olefin of interest at a sufficiently high purity required to serve as, for example, the material of polyolefins.

In the process of the present invention, it is preferred that the conversion ratio of the primary alcohol or the ether for use as the material be adjusted to 75% or less, more preferably to 70% or less, and still more preferably to 65% or less in order to increase the proportion of the α-olefin in the entire olefin products in the reaction mixture to a degree that does not require multiple distillation or purification processes.

In addition to the olefin products and water, the reaction mixture contains unreacted material (i.e., primary alcohol or ether), amine, and one or two kinds of ether intermediate produced in the reaction.

The ether intermediate produced in the reaction is an ether with the structure R¹-O-R¹ when a primary alcohol with the structure R¹-O-H is used as the material, whereas when an ether with the structure R¹-O-R² is used as the material, two types of ether intermediate, having the structures R¹-O-R¹ and R²-O-R², result. For example, if n-octanol is used as the material, the ether intermediate will be dioctyl ether.

By distilling out the components with relatively low boiling points, such as the olefin products and water, from reacton mixture, the unreacted material and the ether intermediate are collected as a residual solution. Then, at least part of the residual solution can be returned to the reaction system to produce the α-olefin. In the process of the present invention, this procedure helps reduce the amount of waste solution and increase the product yield with respect to the material used.

As implied by the description above, the amine for use with the alumina catalyst in the process of the present invention is preferably one that is readily separable from the intended α-olefin and can thus be collected along with the unreacted material (*i.e.*, primary alcohol or ether) and the ether intermediate. Specifically, the amine is one that has a boiling point higher than the boiling point of the intended α-olefin preferably by 30°C or more, and more preferably by 50°C or more, under atmospheric pressure. Upon use, such amine, along with the unreacted material and ether intermediate, remains in the residual solution that remains after the components with relatively low boiling points, including the olefin products and water, have been distilled out from the reaction mixture. This is advantageous since no further amine needs to be added or mixed when at least part of the residual solution is returned to the reaction system. For example, when n-octanol (bp: 196°C under atmospheric pressure) is used as the material, the product is 1-octene (bp: 121°C under atmospheric pressure) and the ether intermediate is di-n-octyl ether (bp: 287°C under atmospheric pressure). A preferred amine in this case is for example octylamine (bp: 177°C under atmospheric pressure), tributylamine (bp: 216°C under atmospheric pressure) or N,N,N'N'-tetramethyl-1,6-hexamethylenediamine (bp: 210°C under atmospheric pressure).

Not only can the process of the present invention for producing α-olefins yield an α-olefin at a high proportion with respect to the entire olefin products, but it also can maintain the catalyst activity for a prolonged time. Furthermore, the process of the present invention allows the use of the unreacted material and the ether intermediate by-product in the reaction using the same catalyst. As a result, the α-olefin can be produced in an industrially and environmentally advantageous manner.

While the present invention will now be described with reference to Examples, they are intended to be only illustrative and not restrictive and do not limit the scope of the invention in any way.

### Example 1

A 1500mm-long glass tube reactor with the inner diameter of 22mm was filled with 20ml of a γ-alumina catalyst (N613N), manufactured by NIKKI Chemical Co., Ltd., and was heated to 300°C by an electric furnace. n-octanol (bp = 196°C) added with 1% by mass of n-octylamine (bp = 177°C) was fed to the tube reactor under atmospheric pressure at a rate of 52ml/hr (LHSV = 2.6hr⁻¹). During the reaction, the temperature at the center of the catalyst layer was maintained at 300°C. The reaction gas obtained at the outlet of the tube reactor was passed through a condenser to condense and collect the reaction mixture as a solution. Part of the reaction mixture was collected at predetermined time points through the course of the reaction and was analyzed by gas chromatography. The reaction was carried out continuously for 150 hours. The results are shown in Table 1 below. As shown, no significant variation was observed in the conversion rate through the course of the reaction.

**TABLE 1**

| Reaction time (hr) | Conversion rate of n-octanol (%) | Selectivity (%) | | Proportion of 1-octene/octenes (%) |
|---|---|---|---|---|
| | | octenes | * ether intermediate | |
| 1 | 56 | 35 | 65 | 99.1 |
| 20 | 49 | 36 | 64 | 98.8 |
| 40 | 48 | 33 | 67 | 98.9 |
| 60 | 48 | 36 | 64 | 99.3 |
| 80 | 49 | 32 | 68 | 99.4 |
| 100 | 48 | 33 | 67 | 99.0 |
| 120 | 47 | 34 | 66 | 99.0 |
| 150 | 49 | 35 | 65 | 99.2 |

| | | | | |
|---|---|---|---|---|
| * di-n-octyl ether | | | | |

### Examples 2 to 4

For each of Examples 2 to 4, reaction was carried out under the same conditions as in Example 1, except that a different amine is added to n-octanol fed to the tube reactor. Two hours after the reaction had been started, part of the reaction mixture was collected and was analyzed by gas chromatography. Amines used in respective Examples are as follows: dimethylaminopyridine (bp = 162°C under 6.7kPa) for Example 2; 1,8-diazabicyclo [5.4.0] undeca-7-ene (bp = 81°C under 79.8Pa) for Example 3; and N,N,N',N'-tetramethyl-1,6-hexamethylenediamine (bp = 210°C under atmospheric pressure) for Example 4. The results are shown in Table 2 below.

**TABLE 2**

| Ex. No. | Conversion rate of n-octanol (%) | Selectivity (%) | | Proportion of 1-octene/octenes (%) |
|---|---|---|---|---|
| | | Octenes | * ether intermediate | |
| 2 | 28.3 | 26.0 | 74.0 | >99.5 |
| 3 | 16.6 | 21.5 | 78.5 | >99.5 |
| 4 | 60.2 | 40.7 | 59.3 | 98.6 |

| | | | | |
|---|---|---|---|---|
| * di-n-octyl ether | | | | |

### Comparative Example 1

In Comparative Example 1, reaction was carried out under the same conditions as in Example 1, except that no amine was added to n-octanol fed to the tube reactor. Two hours after the reaction had been started, part of the reaction mixture was collected and was analyzed by gas chromatography. The results are shown in Table 3 below.

### Comparative Example 2

In Comparative Example 2, reaction was carried out under the same conditions as in Example 1, except that a base-treated alumina was used as the catalyst and no amine was added to n-octanol fed to the tube reactor. The base-treated alumina was prepared in the following manner. A γ-alumina (RN-03, manufactured by MIZUSAWA Chemical Co., Ltd.) was immersed in an aqueous solution of sodium hydroxide with pH 9.9 at a ratio of 5g γ-alumina per 100ml of sodium hydroxide solution at room temperature for 24 hours. The alumina was then washed with water, dried and baked. Two hours after the reaction had been started, part of the reaction mixture was collected and was analyzed by gas chromatography. The results are shown in Table 3 below.

**TABLE 3**

| Comp. Ex. No. | Conversion rate of n-octanol (%) | Selectivity (%) | | Proportion of 1-octene/octenes (%) |
|---|---|---|---|---|
| | | octenes | * ether intermediate | |
| 1 | 93.4 | 97.5 | 2.5 | 82.0 |
| 2 | 46 | 67.9 | 32.1 | 97.0 |

| | | | | |
|---|---|---|---|---|
| * di-n-octyl ether | | | | |

As seen from the results of Comparative Examples 1 and 2, the proportion of 1-octene to the entire octene products was significantly reduced when no amine was added, leading to the industrially unfavorable results. Although the use of the base-treated alumina increased the proportion of 1-octene to the entire octene products in Comparative Example 2 as compared to Comparative Example 1, the increase was still industrially insufficient.

### Example 5

In Example 5, reaction was carried out under the same conditions as in Example 1 except that n-octanol (bp = 196°C) added with 1% by mass of dimethylaminopyridine (bp = 162°C under 6.7kPa) was fed to the tube reactor under atmospheric pressure at a rate of 18mL/hr (LHSV = 0.9 hr⁻¹). Two hours after the reaction had been started, part of the reaction mixture was collected and was analyzed by gas chromatography. The results are shown in Table 4 below.

### Example 6

In Example 6, reaction was carried out under the same conditions as in Example 1 except that n-octanol (bp = 196°C) added with 1% by mass of 1,8-diazabicyclo [5.4.0] undeca-7-ene (bp = 81°C under 79.8Pa) was fed to the tube reactor under atmospheric pressure at a rate of 26mL/hr (LHSV = 1.3 hr⁻¹). Two hours after the reaction had been started, part of the reaction mixture was collected and was analyzed by gas chromatography. The results are shown in Table 4 below.

### Example 7

In Example 7, reaction was carried out under the same conditions as in Example 1, except that the tube reactor was heated to 280°C by an electric furnace and n-octanol (bp = 196°C) added with 1% by mass of N,N,N',N'-tetramethyl-1,6-hexamethylenediamine (bp = 210°C) was fed to the tube reactor under atmospheric pressure at a rate of 52mL/hr (LHSV = 2.6 hr⁻¹) with the temperature at the center of the catalyst layer maintained at 280°C through the course of the reaction. Two hours after the reaction had been started, part of the reaction mixture was collected and was analyzed by gas chromatography. The results are shown in Table 4 below.

**TABLE 4**

| Ex. No. | Conversion rate of n-octanol (%) | Selectivity (%) | | Proportion of 1-octene/octenes (%) |
|---|---|---|---|---|
| | | octenes | *ether intermediate | |
| 5 | 58.0 | 61.4 | 38.6 | 98.5 |
| 6 | 68.6 | 73.3 | 26.7 | 97.4 |
| 7 | 17.5 | 31.3 | 68.7 | >99.5 |

| | | | | |
|---|---|---|---|---|
| * di-n-octyl ether | | | | |

### Example 8

In Example 8, reaction was carried out under the same conditions as in Example 1, except that methyl octyl ether was used in place of n-octanol. As shown, the conversion rate of methyl octyl ether was 25.0%, with the selectivity for entire octene products being 72%. The proportion of 1-octene to the entire octene products was 98.5%.

### Example 9

In Example 9, reaction was carried out under the same conditions as in Example 1, except that a residual solution remaining after octenes had been removed by simple distillation of the reaction mixture obtained in Example 1 was used as the material (composition: 67 mass % n-octanol, 32 mass % dioctyl ether, 1 mass % n-octylamine). The composition of the resulting reaction mixture was as follows: 43.7 mass % n-octanol, 26.0 mass % di-n-octyl ether and 30.3 mass % octenes. The proportion of 1-octene to the entire octene products was 98.5%. These results indicate that the unreacted material and the ether intermediate separated and collected from the reaction mixture can also serve as an efficient material for the reaction.

### Example 10

In Example 10, reaction was carried out under the same conditions as in Example 1, except that n-nonanol was used in place of n-octanol. The conversion rate of n-nonanol was 38.5%, with the selectivity for entire nonene products being 38.9%. The proportion of 1-nonene to the entire nonene products was 98.7%.

### Example 11

In Example 11, reaction was carried out under the same conditions as in Example 1, except that n-decanol was used in place of n-octanol. The conversion rate of n-decanol was 40.2%, with the selectivity for entire decene products being 39.5%. The proportion of 1-decene to the entire decene products was 98.6%.

## Claims

1. A process for producing an α-olefin through elimination reaction of a primary alcohol or an ether, **characterized in that** an alumina catalyst is used in the presence of an amine.

2. The process according to claim 1, wherein an ether intermediate produced in the reaction, along with unreacted primary alcohol or ether, is separated and collected from reaction products, and at least part of the collected ether intermediate, and unreacted primary alcohol or ether is returned to the reaction system.

3. The process according to claim 1 or 2, wherein the amine is one having a boiling point higher than that of the α-olefin product by as much as 30°C or more under atmospheric pressure.

4. The process according to any one of claims 1 to 3, wherein the primary alcohol is n-octanol.
